# EUROPEAN PATENT SPECIFICATION

(11) **EP 1 917 236 B1**
(45) Date of publication and mention of the grant of the patent: **18.08.2010**
(21) Application number: 06700630.4
(22) Date of filing: 12.01.2006
(51) Int. Cl.: C07C 255/00

(54) **CHROMOPHORIC POLYMER**
CHROMOPHORES POLYMER
POLYMERE CHROMOPHORE

(30) Priority: 20.07.2005 US 700794 P
(43) Date of publication of application: 07.05.2008
(73) Proprietor: Mempile Inc., Wilmington, DE 19801 (US)
(72) Inventor: SHIPWAY, Andrew, N., 92542 Jerusalem (IL); GREENWALD, Moshe, 62999 Tel-aviv (IL); JABER, Nimer, 90845 Abu Ghosh (IL)
(74) Representative: Diehl & Partner GbR
(86) International application number: PCT/IL2006/000054
(87) International publication number: WO 2007/010519

(56) References cited:
- WO-A-03/070689
- PATENT ABSTRACTS OF JAPAN vol. 013, no. 315 (C-619), 18 July 1989 (1989-07-18) & JP 01 100171 A (YAMAMOTO CHEM INC; others: 01), 18 April 1989 (1989-04-18)

## Description

### FIELD OF THE INVENTION

This invention relates to additives to polymer matrices that can serve as an optical storage media.

### BACKGROUND OF THE INVENTION

In three-dimensional optical storage, the medium is generally an organic material, which contains chromophores (the molecular data storage component) embedded in a matrix (see WO 01/73,769 and US 5,268,862). Photochromic media consists of chromophores which upon appropriate photochemical excitation, undergo a change of state (e.g. isomerization). Such a change of the chromophore's state permits the inscription ("writing") of data. The matrix which may be polymeric (as described in WO 03/070,689) provides the required mechanical properties to the media, and ideally should be essentially inert and not interfere with the optical processes in a negative way. However, the sensitivity of the medium to storage and retrieval of data is nonetheless influenced by the properties of the matrix. Any photochemical process is to some extent dependant on its microenvironment, for example through a solvatochromic effect in a simple case. In optical data storage, however, the effect of the microenvironment is even greater. For a chromophore to switch between the two forms that represent different data states, it must undergo a chemical transformation, which may require a volume of reaction to permit a reaction transition state. The volume of reaction means that the rate of reaction (and consequently the sensitivity of the media) is highly dependant on the free volume and viscosity of the microenvironment of the chromophore. Likewise, the existence of a reaction transition state suggests that the rate of reaction can be highly dependant on the chemical composition of the microenvironment because of interactions between the transition state and the microenvironment which may lower the transition state energy.

While the polymer matrix could potentially be modified in order to optimize the rate of reaction for the data writing process in an optical media, this can be detrimental to the lifetime of the data itself. In a matrix that generally eases the conversion of one form of the data storage chromophore into the other can allow this process to happen, such conversion can also occur spontaneously, or during data retrieval which may also involve the photochemical excitation of the chromophore. Ideally, the microenvironment of the chromophore should therefore be engineered not only to optimize the rate of chromophore conversion during the writing of data, but also to minimize it during the storage of the media and the readout of data.

### SUMMARY OF THE INVENTION

The present invention provides polymer matrices intended for use as chromophoric optical media, particularly for data storage, where the matrices comprise the polymer bearing the active chromophoric groups together with aromatic additives. It was found in accordance with the invention that such additives increase the sensitivity of the chromophoric groups to undergoing an irradiation induced changes in their state, e.g. a switch between *cis* and *trans* configurations, between spiropyran and merocyanine forms, between the "open" and "closed" forms of diarylethenes and fulgides, or between the two forms of phenoxynaphthacene quinines, and hence an increase in their sensitivity to the inscription ("writing") of the data. This is likely achieved through changes in the microenvironment of the chromophoric groups induced by said additives.

Thus the present invention is directed to an acrylic polymer matrix comprising active chromophoric groups and additives, the additives being aromatic compounds. The aromatic compounds are phenyls substituted with one or more substituents independently selected from:
(i) halogens;
(ii) C₁₋₄ alkyl esters of carboxylic acid or their amides, particularly such esters or amides of C₁-C₆ carboxylic acids, exemplary amides being C₁₋₄ alkylamides;
(iii) -OR, -SR or -C(=O)R, R being an C₁₋₆ alkyl group;
(iv) -O-(CH₂)ₙ-O-C(=O)C(X)=CH₂ where X is CH₃ or H and n is 1-6;
(v) the group -O-C(=O)C(X)=CH₂.

The active chromophoric groups in the matrices of the invention may be pendant groups on the polymeric backbone or may be co-monomers such that the polymer is a co-polymer comprising a polymerizable active chromophore monomer co-polymerized with a monomer.

The substituted phenyl compounds may be doped additives (namely added to the polymer matrix as free compounds) or may be substituents on monomers or co-monomers that are used for production of the matrix. The terms "additive" will be used herein to refer to such compounds both as free compounds or as such substituents.

The substituted phenyl compounds are preferably selected from:
3,4,5-trichlorobenzene;
diethylphthalate;
2-Methyl-acrylic acid 3-(3,4-dichloro-phenoxy)-propyl ester;
5-[3-(2-Methyl-acryloyloxy)-propoxy]-isophthalic acid diethyl ester;
2-Methyl-acrylic acid 3,4-dichloro-phenyl ester;
2-Methyl-acrylic acid 3-(4,6-dichloro-phenoxy)-propyl ester;
2-Methyl-acrylic acid 3-(2,4,6-trichloro-phenoxy)-propyl ester;
2-Methyl-acrylic acid 3-(4-bromo-phenoxy)-propyl ester;
2-Methyl-acrylic acid 4,6-dichloro-phenyl ester;
2-Methyl-acrylic acid 2,4,6-trichloro-phenyl ester;

Preferably, the polymer is a (meth)acylate-based polymer wherein the active chromophoric groups are stilbene derivatives of the following formula (I):

Ar¹C(R¹)=C(R²)Ar² (I)

wherein Ar¹ and Ar² are phenyl groups optionally independently substituted with one or more groups selected from -C₁₋₆alkyls, -OC₁₋₆alkyl, -SC₁₋₆alky; and, -C₁₋₆OH, thiols and their salts, NR'R", R' and R" being independently hydrogen or C₁₋₆alkyl; R¹ and R² are substituents selected from nitriles being a -(CH₂)ₙCN n being 0, 1 or 2, halides, C₁₋₆COOH, their C₁₋₄ alkyl esters, or a nitro compound selected from - (CH₂)ₙNO₂, n being 0, 1 or 2.

C₁₋₆alkyls may be straight or branched alkyls, preferably a methyl, ethyl, propyl, isopropyl, butyl, sec-butyl or tert-butyl as well as pentyl groups; the nitrile is preferably a -CN group and the nitro compound is preferably an NO₂ group.

A polymerizable active chromophore monomer useful in accordance with the invention is preferably a compound of the following formula (II):

Ar¹(R¹)C=C(R²)Ar²-M (II)

wherein Ar¹, Ar², R¹ and R² are as defined above and M is a polymerizable monomeric moiety. Specific example of M are acrylic monomers such as methylmethacrylate (MMA) and methylacrylate (MA) derivatives.

Exemplary photochromic-modified monomers are those of the following formula (III): 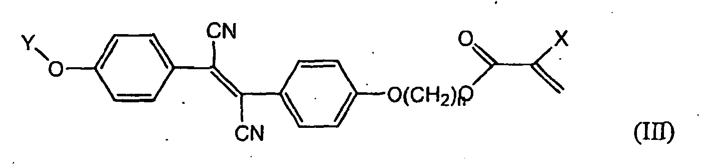 wherein X and Y are as defined above.

Particular examples are polymerizable active chromophore monomers of the following formula (IV) and (V) (also referred to herein as "eMMA" and "eAA", respectively):

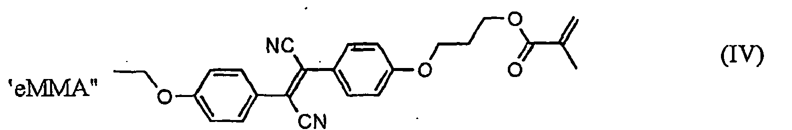

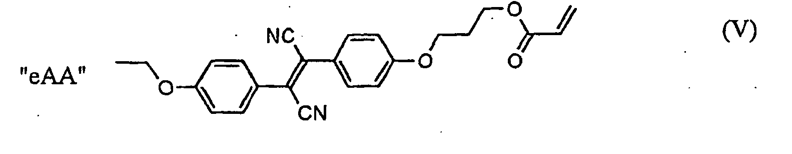

The active chromophoric groups as well as said additives are typically bound to monomeric groups and thereby become bound to the polymeric matrix. The monomeric group is preferably a group that it compatible for copolymerization with (meth)acrylates, for example acrylate, alkylacrylate, styrene or maleimide. A specific example is methylmethacrylate (MMA) or methylacrylate (MA). Such a polymer typically comprises between 5 to 30 wt% of chromophoric groups, 50-65% MMA and 10-20 wt% of a phenyl substituted compound.

The invention is further directed to a photochromic medium for data storage comprising a matrix comprising active chromophoric groups and said additives.

The invention is yet further directed to a 3-dimensional optical data storage comprising a photochromic medium comprising a matrix with active chromophoric groups and with said additives.

The invention is yet further directed to a method for facilitating the inscription of data in a 3-dimensional optical data storage and decreasing loss of data upon storage or subsequent reading of the data, comprising use of a photochromic medium comprising a matrix with active chromophoric groups and with said additives

In accordance with an embodiment of the invention, the photochromic medium is prepared with a high concentration of the active chromophoric groups. The photochromic medium according to one embodiment being characterized in that the active chromophore is included in the polymeric substance at a concentration greater than 10% (wt%). The non linear positive increase in writing sensitivity with increased concentration of active chromophores is termed cooperativity. Such copperativity is described in co-pending, co-owned application entitled "Improved disks for data storage". The addition of the above-mentioned additives causes an enhancement of the coopertivity.

### BRIEF DESCRIPTION OF THE DRAWINGS

In order to understand the invention and to see how it may be carried out in practice, a preferred embodiment will now be described, by way of non-limiting example only, with reference to the accompanying drawings, in which:
**Fig. 1** illustrates the decrease in laser pulses required to "write" a data point when the photochromic medium comprises aromatic additives according to the invention.
**Fig. 2** illustrates that the relative "graying" rate (rate of loss of written information) in photochromic medium comprising aromatic additives according to the present invention is reduced.

### DETAILED DESCRIPTION OF EXEMPLARY EMBODIMENTS

As mentioned above, the present invention provides a chromophoric optical medium, where the medium is a polymeric medium preferably based on acrylic or acrylic-compatible monomers, e.g. (alkyl)acrylates, styrenes, and maleimides, the polymer matrix comprising active chromophoric groups and said additives, being substituted phenyl compounds. The photochromic media consists of chromophores which upon a photochemical excitation undergo a change of state (e.g. isomerization). Such a change may give rise to the inscription (or erasure) of data. The additives induce changes in the microenvironment of the chromophoric groups and increase their sensitivity to the inscription of the data. It was found that the substituted phenyl additives increase the sensitivity of photochromic optical media to data writing, substantially without causing loss in data stability and substantially without having a significant effect on the solubility of chromophores.

Without wishing to be bound by theory, the substituted phenyl additives appear to ease the "writing" of the data by a neighboring effect, where neighboring substituted phenyl additives affect the active chromophore increasing the rate of "writing". The microenvironment of the chromophoric active groups may be visualized as surrounded by appropriate substituted phenyl additives easing of and aiding to molecular freedom of movement. The photochromic medium is a polymer where the active chromophoric groups are either part of the polymer backbone or are bonded as pendant groups. The substituted phenyl additives may be part of the polymers backbone or are uniformly distributed within the polymer matrix.

The substituted phenyl additives are substituted by substituents selected from halogens, such F, Cl, Br, I; C₁₋₄ alkyl esters of carboxylic acid or their amides; -OR, - SR or -C(=O)R, R being an C₁₋₆ alkyl group; the group -O-(CH₂)ₙ-O-C(=O)C(X)=CH₂ where X is CH₃ or H and n is independently 1-6, or the group-O-C(=O)C(X)=CH₂ or their mixtures. The halogens are preferably Cl, Br or I. The alkyl esters of the carboxylic acids which may be fluorinated are preferably methyl, ethyl or propyl groups. X is preferably a methyl group and n is preferably 0, 1, 2, 3 or 4.

Such a microenvironment gives the polymer chains appropriate mobility and an appropraite polarity. The added substituted phenyl additives may have some interaction with the chromophores, which are groups that have Hildebrand solubility parameters close to that of the chromophore, and consequently may have some interaction with the chromophores on the basis of their compatibility. The added substituted phenyl groups are "spacious" groups that produce disturbance in the matrix when they move. Appropriate mobility or freedom of movement is a result of additive-aided disturbance in the otherwise movement-resistant local environment when a moiety moves.

The added substituted phenyl groups are doped additives or are co-monomers, thus forming eventually part of the polymeric matrix. In the case of doped additives, these compounds are simply added to the composition. Compounds suitable for doping must have a low volatility so that the resulting media is stable with no migration of the added groups. The polymer backbone may be engineered by substituting the polymerizable groups attached to the chromophore and/or other co-monomers with other polymerizable groups compatible with the polymer, such as acrylate or methacrylate. The structures of the added substituted phenyl groups maximize writing performance without lowering of chromophore solubility.

Accordingly, disks were prepared by the bulk copolymerization of MMA at 60°C with 10 wt% of the chromophore-bound monomer "eMMA" (formula (IV) below) and dilauryl peroxide (2 wt%) as an initiator. (It is possible to replace the eMMA of formula (IV) with the "eAA" of formula (V)

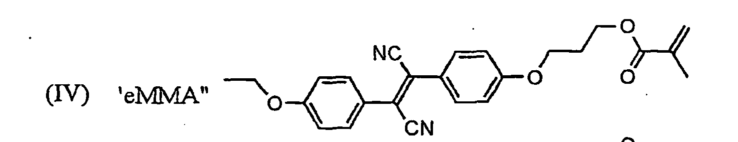

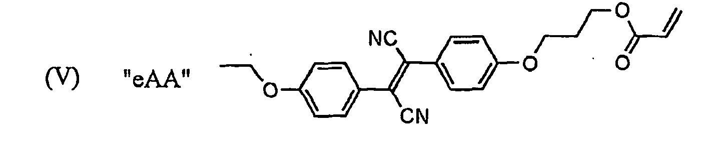

Various substituted phenyl groups were also included in the mixture in order to demonstrate their ability to enhance the data storage properties of the media. Data storage and retrieval were tested by measuring:
(i) the power required to write data (in Watts required to make a 10% modulation mark using a 17 ns laser pulse at 671 nm),
(ii) the relative strength of the signal received when reading the disk (fluorescence), and
(iii) the relative rate at which data is destroyed by continuously reading it.
The values reported are relatives to a regular sample that doesn't posses any additive.

The values reported are relative to a regular sample that does not posses any additive.

The data measured for disks with and without certain additives are presented in Table 1. While large values are good for (ii), small numbers are good for (i) and (iii).

**Table 1**

| **Matrix additives** | **(i) Peak power in writing** | **(ii) Relative signal strength** | **(iii) Graying** |
|---|---|---|---|
| No additives | 63 | 1.13 | 0.99 |
| Diethylphthalate (10%) | 56 | 1.28 | 0.87 |
| 1,2,3-Trichlorobenzene (10%) | 57 | 1.13 | 1.30 |
| N,N-Dibutylformamide (10%)* | 69 | 0.98 | 1.39 |

| | | | |
|---|---|---|---|
| * Additive not according to the invention | | | |

It is clear that relative to the disk containing no additives, the chlorinated aromatic compound and the ester of the aromatic diacid reduce the writing power, have no or a small positive effect on the signal strength, and have little effect on the data stability. It was found that the ester could easily be introduced into a disk at 20 wt% concentration, and the chlorinated compound at more than 30 wt% concentration. In contrast, the nonaromatic amide has a negative effect on all three parameters: it requires the highest writing power, produces the least signal, and has the most volatile data. Thus, as explained, the incorporation of the additives give improved results in lower required power and higher signal strength. However, such incorporation may give rise to various problems associated with the presence of additives in polymers. The option of using the substituted phenyl groups as covalently linked to the polymer matrix renders them unable to migrate. Such a use enables selectivity and specificity where the added groups can be selectively used in the appropriate part of a polymer. In case the substituted phenyl additive is a co-monomer to the polymer, it is structurally represented as (VI),

P-X-Ar-Rn (VI)

where P is a polymerizable group (e.g. MMA ,MA (methacrylate)) intended for radical or anionic polymerization, X being the spacer -O(CY₂)ₙ, Y being independently H, C₁₋₄alkyl, halogen or aryl, preferably H or C₁₋₄alkyl; n being 0-8, preferably, 0, 1, 2, 3 ,4, 5 or 6; Ar-Rn is the substituted phenyl compound according to the present invention (Rn being the one or more substituents as described above).

In particular, the polymerizable group may have the following structures:

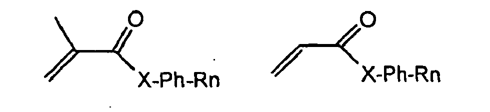

Specific compounds are (VII)-(XIX) given in Table 2.

**Table 2**

| Structure | Name | Structure no. |
|---|---|---|
| | | |
| 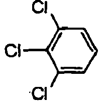 | 1,2,3-Trichlorophenol | *VII |
| 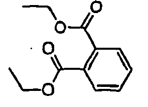 | Diethyl phthalate | *VIII |
| 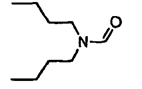 | Dibutyl formamide | *IX |
| 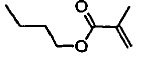 | Butyl methacrylate | X |
| 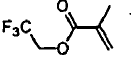 | 1,1,1-trifluoroethyl methacrylate | XI |
| 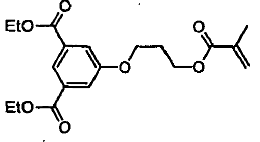 | | XII |
| 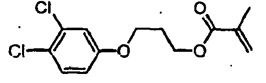 | | XIII |
| 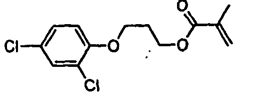 | | XIV |
| 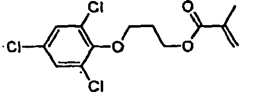 | | XV |
| 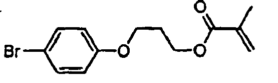 | | XVI |
| 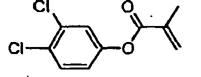 | | XVII |
| 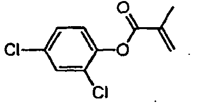 | | XVIII |
| 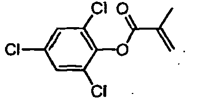 | | XIX |

| | | |
|---|---|---|
| *Compounds VII-IX are commercially available. | | |

In order to demonstrate the efficacy of the additives, disks with (XII), (XIII) and some other additives, as well as disks with without the additives were synthesized. The power required to write data marks in each sample were measured (a low number is preferable). These results are given in Table 3 in decreasing order of sensitivity.

**Table 3**

| **Disk no.** | **eMMA conc., wt%** | **Disk additives** | **Writing power, W (75 ns)** |
|---|---|---|---|
| 05-0069-02 | 30% | 15% (XIII)* | 14.1 |
| 05-0069-03 | 30% | none | 14.6 |
| 05-0068-02 | 20% | 20% Trichlorobenzene* | 14.9 |
| 05-0068-01 | 20% | 20% (XIII)* | 15.7 |
| 05-0069-01 | 20% | 20% (XII)* | 17.0 |
| MT20% | 20% | none | 18.1 |
| 05-0067-01 | 10% | 10% (XIII)* | 20.0 |
| 05-0037-02 | 10% | 10% Trichlorobenzene* | 20.6 |
| 05-0042-01 | 10% | 20% diethylphthalate* | 22.5 |
| 05-0021-01 | 10% | none | 24.3 |
| 05-0053-03 | 10% | 42% butyl methacrylate | 27.0 |
| 05-0061-02 | 10% | 25% methyl acrylate | 28.4 |

| | | | |
|---|---|---|---|
| * Additives according to the invention | | | |

One may observe that, generally speaking, samples containing a larger amount of eMMA require lower writing powers. However, looking within the set of disks with each concentration of eMMA it is clear that additives in accordance with the invention improve the properties of the medium. All the disks containing the additives of interest perform better than the comparable disks containing no additives. It is seen that additives which are not substituted phenyl groups do not give rise to improvement in disk properties.

In order to further demonstrate the efficacy of several of the compounds at slightly different power and duration of irradiation, disks with and without the substituted phenyl groups were synthesized. The power required to write data marks in each sample were measured (a low number is preferable). These results are given in Table 4. Generally, a chromophoric medium containing 20 wt% chromophore, 65% MMA and 15% a substituted phenyl group were prepared. The number of laser pulses (60 ns, 4.8 W) required to create a data mark (5% modulation) was recorded for each prepared chromophoric medium, along with the relative rate at which data is destroyed during reading ("graying"). The results are given in Table 4.

**Table 4**

| **Additive** | **Pulses to write** | **Relative graying** |
|---|---|---|
| None (MMA) | 50 | 1.20 |
| 15% XIII | 41 | 1.10 |
| 15% XV | 32 | 1.50 |
| 15% XI | 42 | 1.70 |
| 15% XIX | 36 | 0.75 |
| 15% XVI | 41 | 1.30 |

It is seen that in all cases the additives reduce the number of pulses required to write data. However, in the case of XI (which is not an additive according to the present invention) this is accompanied by a significant increase in the "graying" rate. This demonstrates a general softening of the chromophore's microenvironment, leading to generally faster isomerization. The case of XIX is markedly different. In this case the sensitivity to writing is increased (even more than it is for XI), but the "graying" rate is actually decreased. In other words, isomerization is faster than the reference during "writing" and slower than the reference during "reading". This phenomenon can be likely explained as the consequence of micronenvironment-induced effects. This additive is bulky, causing the polymer to be hard so that the "graying" rate is low. However, upon the intense irradiation of the "writing" laser, the bulk and stiffness of this monomer allow the matrix to transmit movements such that isomerization becomes much faster.

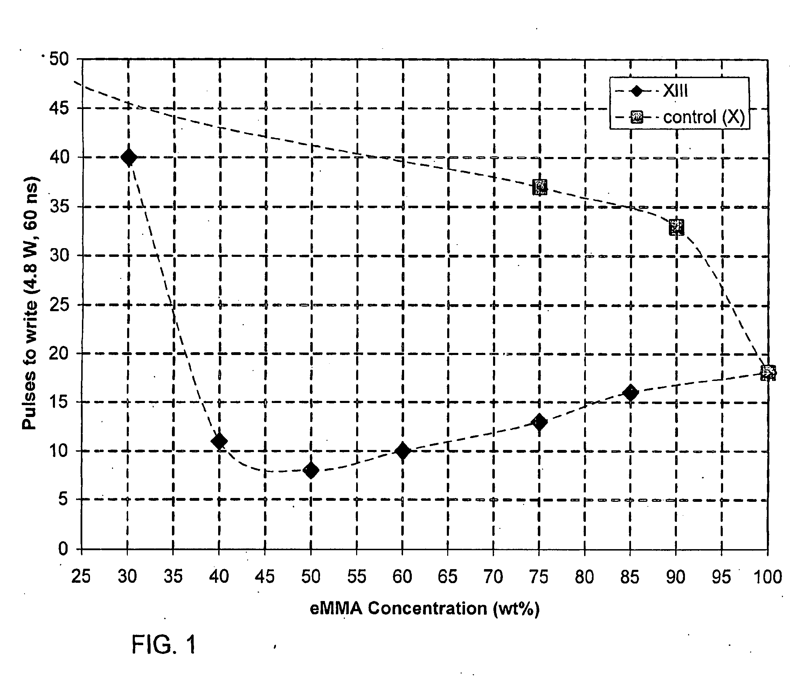

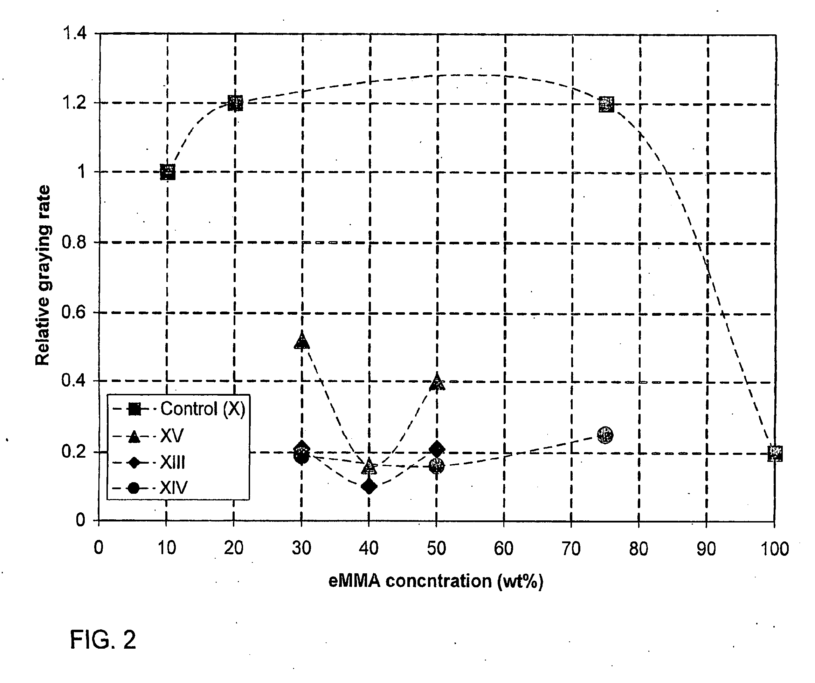

In **Figs. 1** and **2**, the effect of the addition of the substituted phenyl groups which ease the "writing" data is further demonstrated. A series of samples containing various ratios of eMMA and other monomers were produced by copolymerizing eMMA with another monomer in various ratios to produce samples that were examined as mentioned above (power of irradiation to produce a signal was measured). The plots given in **Figs. 1** and **2** show that the number of laser pulses required to "write" a data point and the relative "graying rate" vary with the concentration ratio. The x axis in each case gives the concentration (in wt%) of eMMA. The remainder is the monomer as indicated in the series name. 100% eMMA is a special point, since there is no additive present, and therefore all lines converge to the same point.

Turning to **Fig. 1** (showing the sensitivity to writing for samples containing XIII), the control series (with butyl(meth) acrylate (BMA) as the co-monomer) shows the expected curve shape for a cooperative concentration effect as discussed above. As the eMMA concentration rises, the material becomes progressively more sensitive. However, the additive XIII behaves rather differently. First, for the same concentration of eMMA these samples have a much greater sensitivity. This demonstrates how this monomer alters the microenvironment to enhance the cooperative effect. Importantly, it can be seen that as the eMMA concentration approaches 100% the sensitivity decreases. This is attributed to the decreasing amount of XIII in the matrix, demonstrating that XIII enhances write sensitivity beyond even what a homopolymer of eMMA is capable of. This effect is different from coopertivity. This is also an example of how the engineering of the thermophysical properties of the matrix can lead to optimization. DSC measurements of the chromophoric medium shown in Fig. 1 show that as the proportion of XIII in the matrix increases, the glass transition temperature of the material decreases. The change is approximately linear between the 100% eMMA sample (Tg ∼70°C) and the 50% eMMA samples (Tg ∼50°C). The lower Tg of the matrices containing more XIII means that they can allow molecular movements more easily under the ATE, helping contribute to their higher sensitivity towards "writing". The "graying" rate of binary copolymers of eMMA with XV, XIII, or XIV are all very low compared to that of the control series, as shown in **Fig. 2****.** This result is explained by the "bulkyness" of the added substituted phenyl groups contained in the monomers. Once again, this demonstrates how a correctly engineered matrix can both enhance sensitivity to writing and decrease destructive reading "graying" at the same time. Particularly of note is the fact that the maximum activity of the matrix to writing enhancement and to graying inhibition both occur at around the same composition (40-50% eMMA).

### Examples

In general, compounds of formulae (XII) and (XIII) can be synthesized by the Williamson reaction of 3-bromopropyl methacrylate with 5-hydroxy-diethylisophthalate (for (XII)) or 3,4-dichlorophenol (for (XIII)). Other examples may also be synthesized by the Williamson reaction or the reaction of the relevant phenol with a carboxylic acid anhydride. For example, 2,4,6-trichlorophenyl methacrylate is synthesized in 70% yield by the slow addition of 50 mL triethylamine to a mixture of 50 g 2,4,6-trichlorophenol, 50 mL acetone and 50 mL methacrylic anhydride. The acetone is removed under vacuum, 200 mL of petroleum ether is added, then the solution is filtered through 5 mm siilca gel and left at -20 C for the product to crystallize. The product is collected by filtration.

### Example I: Synthesis of (XIII):

To a double jacket 3 liter reactor, equipped with a mechanical stirrer and condenser, 3,4-dichlorophenol (40 g), 3-bromopropyl methacrylate (56 g), potassium carbonate (336 g) and MeCN (1500 g) were introduced. Argon was bubbled while stirring for 15 min., then the reactor was heated to reflux under argon for 3h. The reaction mixture was filtered, then the filtrate was evaporated under vacuum. 1 liter petroleum ether was added, the solution become cloudy, then filtration and evaporation of the petrol ether by evaporator led to a white solid. The solid was washed twice with 100 ml H₂O:CH₃OH (1:2) then dried under vacuum to yield 31 g of product in a purity of 98.3%. The washed solid was dissolved in 100 ml of CHCl₃: petroleum ether (80:20) and filtered through 35 g silica (70-370 mesh). The silica was then washed with an additional 200 ml CHCl₃: petroleum ether (80:20). Evaporation of the combined solvents led to a 30g of product (99.3% pure). The petroleum ether phase from the first washing was evaporated and the resulting liquid was filtrated through silica gel to produce another 24 g of product.

### Example II: Synthesis of compounds XII-XVI (and analogs):

3-bromopropyl methacrylate and the appropriate phenol are reacted according to the following procedure: The phenol (1 mol) is dissolved in MeCN (300 mL), then anhydrous potassium carbonate (2 mol) and 3-bromopropyl methacrylate (1.1 mol) are added. The stirring mixture is heated to reflux for 4 hours, then the MeCN is removed by distillation. After cooling, petroleum ether (500. mL) is added to the resulting slurry, which is then filtered through a plug of 5 mm silica gel. The solids are washed with a further 500 mL of petroleum ether, then the combined petroleum ether fractions are gradually cooled until the product crystallizes (-20 to -50°C, depending on the compound). The product is collected by filtration, then is dried under vacuum to give an analytically pure compound at 50-90% yield.

### Example III: Process for preparing compounds XVII-XIX (and analogs):

Methacrylic anhydride and the appropriate phenol are reacted according to the following procedure: The phenol (1 mol) is dissolved in a mixture of acetone (150 mL) and methacrylic anhydride (1.2 mol). Triethylamine (1.1 mol) is added at ambient temperature over 15 minutes, then the reaction is allowed to stand for 15 minutes. The acetone is remove by vacuum distillation and petroleum ether (300 mL) is added. The resulting mixture is extracted with (i) 300 mL water, (ii) 300 mL 0.5 M NaOH, (iii) 300 mL brine, and is then dried over magnesium sulfate. The solution is then filtered through a plug of 5 mm silica gel and the solid is washed with petroleum ether (300 mL). The combined petroleum ether fractions are gradually cooled until the product crystallizes (0 to -50°C, depending on the compound). The product is collected by filtration, then is dried under vacuum to give an analytically pure compound at 50-90% yield.

## Claims

1. A polymer matrix comprising active chromophoric groups and aromatic compounds selected from phenyl substituted compounds having one or more substituents independently selected from:
(i) halogen;
(ii) C₁₋₄alkyl esters of carboxylic acid or their amide;
(iii) -OR, -SR or -C(=O)R, R being an C₁₋₆alkyl group;
(iv) -O- (CH₂)ₙ-O-C (=O) C (X) =CH₂; wherein n is independently 1-6; and
(v) the group -O-C (=O) C (X) =CH₂,
wherein X is CH₃ or H.

2. A polymer matrix according to claim 1, wherein said aromatic compounds are doped additives.

3. A polymer matrix according claim 1 or 2, wherein said aromatic compounds are substituents on monomers or co-monomers.

4. A polymer matrix according to any one of claims 1-3, wherein the phenyl substituted compounds are selected from:
3,4,5-trichlorobenzene;
diethylphthalate;
2-Methyl-acrylic acid 3-(3,4-dichloro-phenoxy)-propyl ester;
5-[3-(2-Methyl-acryloyloxy)-propoxy]-isophthalic acid diethyl ester;
2-Methyl-acrylic acid 3,4-dichloro-phenyl ester;
2-Methyl-acrylic acid 3-(4,6-dichloro-phenoxy)-propyl ester;
2-Methyl-acrylic acid 3-(2,4,6-trichloro-phenoxy)-propyl ester;
2-Methyl-acrylic acid 3-(4-bromo-phenoxy)-propyl ester;
2-Methyl-acrylic acid 4,6-dichloro-phenyl ester; and
2-Methyl-acrylic acid 2,4,6-trichloro-phenyl ester.

5. A polymer matrix according to any one of claims 1-4, wherein said active chromophoric groups are pendant groups o the polymer.

6. A polymer matrix according to any one of claims 1-4, wherein said active chromophoric groups are part of the polymer backbone.

7. A polymer matrix according to any one of claims 1-6 being a polymethylmethacrylate based polymer, wherein the active chromophoric groups are stilbene derivatives of the following formula:
Ar¹C(R¹)=C(R²)Ar²
wherein Ar¹ and Ar² are phenyl groups, optionally independently substituted with one or more groups selected from -C₁₋₆alkyls, -OC₁₋₆alkyl, -SC₁₋₆alkyl; and, -C₁₋₆alkylOH, thiols and their salts, NR' R" , R' and R" being independently selected from hydrogen or C₁₋₆alkyl; R¹ and R² are substituents selected from -(CH₂)ₙCN, n being 0, 1 or 2, halide, carboxylic acid and their esters, or a nitro compound of the general formula - (CH₂) ₙNO₂, n being 0, 1 or 2.

8. A photochromic medium comprising a polymer matrix according to any one of claims 1-7, wherein the chromophoric groups are active chromophore monomer having the following formula:
Ar¹(R¹)C=C(R²)Ar²-M(II)
wherein Ar¹, Ar², R¹ and R² are as defined in Claim 7 and M is a polymerizable monomeric moiety.

9. A photochromic medium according to Claim 8, wherein M is an acrylic monomer.

10. A photochromic medium according to Claim 9, wherein said acrylic monomer is selected from methylmethacrylate and methylacrylate.

11. A photochromic medium according to Claim 10, wherein said active chromophore monomer is of the formula: 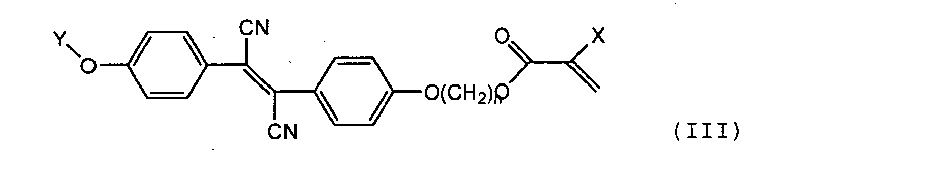 wherein X is methyl or hydrogen;
n is an integer between 1 and 6;
Y is hydrogen or a linear or branched alkyl moiety having 1 to 8 carbon atoms optionally substituted with halogens.

12. A photochromic medium according to claim 11, wherein said active chromophore monomer is selected from the formulae:

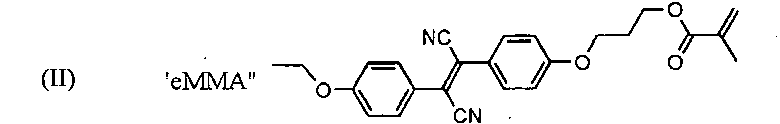

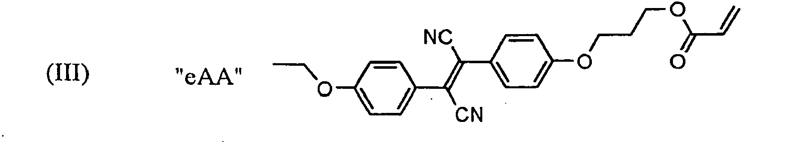

13. A photochromic medium according to any one of Claims 1-12, wherein the active chromophore is included in the polymeric substance at a concentration greater than 5% (wt%).

14. A photochromic medium according to Claim 13, wherein said active chromophore is substantially homogeneously distributed within said photochromic medium.

15. A photochromic medium according to Claim 13 or 14, wherein said active chromophore is substantially non-homogeneously distributed within said supporting substance.

16. A photochromic medium according to Claim 13 or 14, wherein said active chromophore is distributed in discrete portions of high concentration separated by portions devoid of or having insignificant concentration of said active chromophore.

17. A photochromic medium according to Claim 16, wherein said active chromophore is distributed in spaced-apart clusters each formed by said discrete portions of high concentration.

18. A photochromic medium according to any one of claims 13-17, wherein said active chromophore is chemically bound to the polymer.

19. A photochromic medium according to claim 18, wherein said photochromic medium is a co-polymer comprising a polymerizable active chromophore monomer co-polymerized with a monomer.

20. A photochromic medium according to claim 18, wherein said photochromic medium is a polymer comprising a polymerizable active chromophore monomer.

21. A photochromic medium for data storage comprising a polymer matrix according to any of claims 1-7.

22. A 3-dimensional optical data storage comprising a photochromic medium according to claim 8-21.

23. A method for facilitating the inscription of data in a 3-dimensional optical data storage and decreasing loss of data upon storage or subsequent reading of the data, comprising use of a photochromic medium of claim 21.

## Patentansprüche

1. Polymermatrix umfassend aktive chromophore Gruppen und aromatische Verbindungen ausgewählt aus mit Phenyl substituierten Verbindungen, die einen oder mehrere Substituenten aufweisen, die unabhängig ausgewählt sind unter:
(i) Halogen,
(ii) C₁₋₄-Alkylestern von Carbonsäure oder ihrem Amid;
(iii) -OR, -SR oder -C(=O)R, wobei R eine C₁₋₆-Alkylgruppe ist;
(iv) -O-(CH₂)ₙ-O-C(=O)C(X)=CH₂, wobei n unabhängig 1 - 6 beträgt; und
(v) der Gruppe -O-C(=O)C(X)=CH₂,
wobei X CH₃ oder H ist.

2. Polymermatrix nach Anspruch 1, wobei die aromatischen Verbindungen dotierte Zusatzmittel sind.

3. Polymermatrix nach Anspruch 1 oder 2, wobei die aromatischen Verbindungen Substituenten an Monomeren oder Comonomeren sind.

4. Polymermatrix nach einem der Ansprüche 1 - 3, wobei die mit Phenyl substituierten Verbindungen ausgewählt sind unter:
3,4,5-Trichlorbenzol;
Diethylphthalat;
2-Methylacrylsäure-3-(3,4-dichlorphenoxy)propylester;
5-[3-(2-Methylacryloyloxy)propoxyisophthalsäurediethylester;
2-Methylacrylsäure-3,4-dichlorphenylester;
2-Methylacrylsäure-3-(4,6-dichlorphenoxy)propylester;
2-Methylacrylsäure-3-(2,4,6-trichlorphenoxy)propylester;
2-Methylacrylsäure-3-(4-bromphenoxy)propylester;
2-Methylacrylsäure-4,6-dichlorphenylester; und
2-Methylacrylsäure-2,4,6-trichlorphenylester.

5. Polymermatrix nach einem der Ansprüche 1 - 4, wobei die aktiven chromophoren Gruppen Seitengruppen am Polymer sind.

6. Polymermatrix nach einem der Ansprüche 1 - 4, wobei die aktiven chromophoren Gruppen Teil der Polymerrückgratkette sind.

7. Polymermatrix nach einem der Ansprüche 1 - 6, die ein Polymer auf der Basis von Polymethylmethacrylat ist, wobei die aktiven chromophoren Gruppen Stilbenderivate mit der folgenden Formel:
Ar¹C(R¹)=C(R²)Ar²
sind, wobei Ar¹ und Ar² Phenylgruppen sind, die wahlweise unabhängig durch eine oder mehrere Gruppen substituiert sind ausgewählt unter -C₁₋₆-Alkylen, -OC₁₋₆-Alkyl, -SC₁₋₆-Alkyl; und C₁₋₆-AlkylOH, Thiolen und ihren Salzen, NR'R " , wobei R' und R " unabhängig unter Wasserstoff oder C₁₋₆-Alkyl ausgewählt sind; R¹ und R² Substituenten sind, die unter -(CH₂)ₙCN, wobei n 0, 1 oder 2 beträgt, Halogenid, Carbonsäure und ihren Estern oder einer Nitroverbindung mit der allgemeinen Formel -(CH₂)ₙNO₂, wobei n 0, 1 oder 2 beträgt, ausgewählt sind.

8. Photochromes Medium umfassend eine Polymermatrix nach einem der Ansprüche 1 - 7, wobei die chromophoren Gruppen aktives chromophores Monomer sind, das folgende Formel aufweist:
Ar¹(R¹)C=C(R²)Ar²-M (II)
wobei Ar¹, Ar², R¹ und R² die in Anspruch 7 angegebene Definition aufweisen und M ein polymerisierbare monomere Einheit ist.

9. Photochromes Medium nach Anspruch 8, wobei M ein Acrylmonomer ist.

10. Photochromes Medium nach Anspruch 9, wobei das Acrylmonomer unter Methylmethacrylat und Methylacrylat ausgewählt wird.

11. Photochromes Medium nach Anspruch 10, wobei das aktive chromophore Monomer die Formel 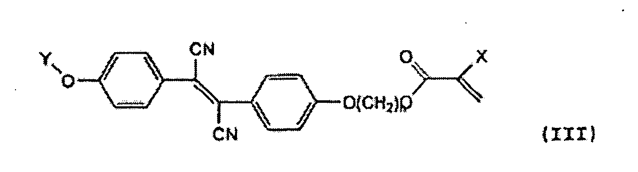 aufweist,
wobei X Methyl oder Wasserstoff ist;
n eine ganze Zahl zwischen 1 und 6 ist;
y Wasserstoff oder ein linearer oder verzweigter Alkylanteil mit 1 bis 8 Kohlenstoffatomen ist, die wahlweise mit Halogenen substituiert sind.

12. Photochromes Medium nach Anspruch 11, wobei das aktive chromophore Monomer unter den Formeln: 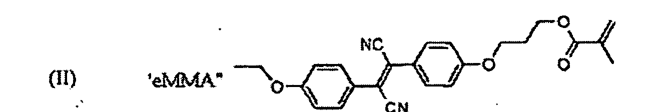 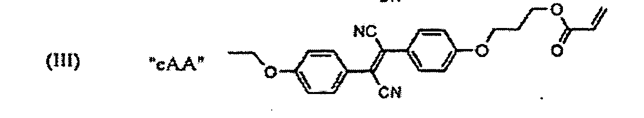 ausgewählt ist.

13. Photochromes Medium nach einem der Ansprüche 1 bis 12, wobei das aktive Chromophor in der polymeren Substanz in einer Konzentration von mehr als 5 % (Gew.-%) enthalten ist.

14. Photochromes Medium nach Anspruch 13, wobei das aktive Chromophor im Wesentlichen homogen innerhalb des photochromen Mediums verteilt ist.

15. Photochromes Medium nach Anspruch 13 oder 14, wobei das aktive Chromophor im Wesentlichen nichthomogen innerhalb der Trägersubstanz verteilt ist.

16. Photochromes Medium nach Anspruch 13 oder 14, wobei das aktive Chromophor in einzelnen Anteilen hoher Konzentration, die durch Anteile getrennt sind, die kein oder eine insignifikante Konzentration des aktiven Chromophors aufweisen, verteilt ist.

17. Photochromes Medium nach Anspruch 16, wobei das aktive Chromophor in beabstandeten Gruppen verteilt ist, von denen jede durch die einzelnen Anteile hoher Konzentration gebildet ist.

18. Photochromes Medium nach einem der Ansprüche 13 - 17, wobei das aktive Chromophor chemisch an das Polymer gebunden ist.

19. Photochromes Medium nach Anspruch 18, wobei das photochrome Medium ein Copolymer ist, das ein polymerisierbares aktives chromophores Monomer umfasst, das mit einem Monomer copolymerisiert ist.

20. Photochromes Medium nach Anspruch 18, wobei das photochrome Medium ein Polymer ist, das ein polymerisierbares aktives chromophores Monomer umfasst.

21. Photochromes Medium für die Datenspeicherung, umfassend eine Polymermatrix nach einem der Ansprüche 1 - 7.

22. 3-Dimensionaler optischer Datenspeicher umfassend ein photochromes Medium nach Anspruch 8 - 21.

23. Verfahren zum Erleichtern des Eintragens von Daten in eine 3-dimensionale optische Datenspeicherung und zum Reduzieren des Verlusts von Daten bei der Speicherung oder dem darauffolgenden Lesen der Daten, umfassend die Verwendung eines photochromen Mediums nach Anspruch 21.

## Revendications

1. Matrice polymère comprenant des groupes chromophores actifs et des composés aromatiques sélectionnés parmi des composés phényl-substitués ayant un ou plusieurs substituants indépendamment sélectionnés parmi :
(i) halogéno ;
(ii) les esters d'alkyle en C₁₋₄ d'un acide carboxylique ou leur amide ;
(iii) -OR, -SR ou -C(=O)R, R étant un groupe alkyle en C₁₋₆ ;
(iv) -O-(CH₂)ₙ-O-C(=O)C(X)=CH₂, n étant compris indépendamment entre 1 et 6 ; et
(v) le groupe -O-C(=O)C(X)=CH₂, dans lequel X représente CH₃ ou H.

2. Matrice polymère selon la revendication 1, dans laquelle lesdits composés aromatiques sont des additifs dopés.

3. Matrice polymère selon la revendication 1 ou 2, dans laquelle lesdits composés aromatiques sont des substituants sur des monomères ou des comonomères.

4. Matrice polymère selon l'une quelconque des revendications 1 à 3, dans laquelle les composés phényl-substitués sont sélectionnés parmi :
• le 3,4,5-trichlorobenzène ;
• le diéthylphtalate ;
• l'ester de 3-(3,4-dichloro-phénoxy)-propyle de l'acide 2-méthyl-acrylique ;
• l'ester diéthylique de l'acide 5-[3-(2-méthyl-acryloyloxy)-propoxy]-isophtalique ;
• l'ester 3,4-dichloro-phénylique de l'acide 2-méthyl-acrylique ;
• l'ester de 3-(4,6-dichloro-phénoxy)-propyle de l'acide 2-méthyl-acrylique ;
• l'ester de 3-(2,4,6-trichloro-phénoxy)-propyle de l'acide 2-méthyl-acrylique ;
• l'ester de 3-(4-bromo-phénoxy)-propyle de l'acide 2-méthyl-acrylique ;
• l'ester 4,6-dichloro-phénylique de l'acide 2-méthyl-acrylique ; et
• l'ester 2,4,6-trichloro-phénylique de l'acide 2-méthyl-acrylique ;

5. Matrice polymère selon l'une quelconque des revendications 1 à 4, dans laquelle lesdits groupes chromophores actifs sont des groupes en pendentif sur le polymère.

6. Matrice polymère selon l'une quelconque des revendications 1 à 4, dans laquelle lesdits groupes chromophores actifs font partie du squelette du polymère.

7. Matrice polymère selon l'une quelconque des revendications 1 à 6, qui est un polymère à base de poly(méthacrylate de méthyle), les groupes chromophores actifs étant des dérivés du stilbène ayant la formule suivante :
Ar¹C(R¹)=C(R²)Ar²
dans laquelle Ar¹ et Ar² sont des groupes phényle, facultativement et indépendamment substitués par un ou plusieurs groupes sélectionnés parmi -(alkyle en C₁₋₆), -O(alkyle en C₁₋₆), -S(alkyle en C₁₋₆) , - (alkyle en C₁₋₆)OH, des thiols et leurs sels, NR'R " , R' et R'' étant indépendamment sélectionnés parmi l'hydrogène ou alkyle en C₁₋₆ ; R¹ et R² sont des substituants sélectionnés parmi -(CH₂)ₙCN, n représentant 0, 1 ou 2, un halogénure, un acide carboxylique, et les esters de ceux-ci, ou un composé nitro de formule générale -(CH₂)ₙNO₂, n représentant 0, 1 ou 2.

8. Milieu photochromique comprenant une matrice polymère selon l'une quelconque des revendications 1 à 7, les groupes chromophores étant un monomère chromophore actif ayant la formule suivante :
Ar¹(R¹)C=C(R²)Ar²-M(II)
dans laquelle Ar¹, Ar², R¹ et R² sont tels que défini dans la revendication 7 et M est un groupe fonctionnel monomérique polymérisable.

9. Milieu photochromique selon la revendication 8, dans lequel M est un monomère acrylique.

10. Milieu photochromique selon la revendication 9, dans lequel ledit monomère acrylique est sélectionné parmi le méthacrylate de méthyle et l'acrylate de méthyle.

11. Milieu photochromique selon la revendication 10, dans lequel ledit monomère chromophore actif a pour formule : 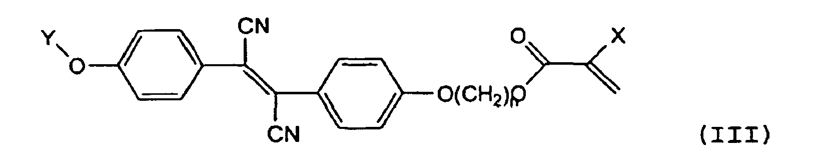 où :
X représente méthyle ou l'hydrogène ;
n est un entier compris entre 1 et 6 ;
Y représente l'hydrogène ou un groupe fonctionnel alkyle linéaire ou ramifié ayant de 1 à 8 atomes de carbone facultativement substitués par des halogènes.

12. Milieu photochromique selon la revendication 11, dans lequel ledit monomère chromophore actif est sélectionné parmi les formules :

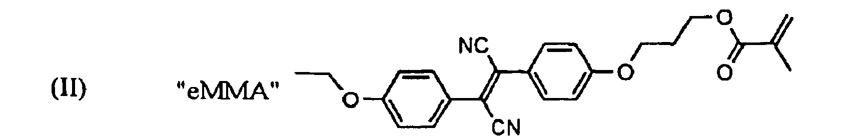

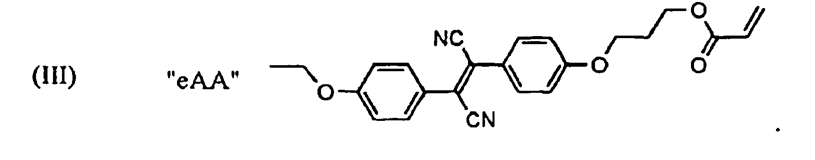

13. Milieu photochromique selon l'une quelconque des revendications 1 à 12, dans lequel le chromophore actif est inclus dans la substance polymérique à une concentration supérieure à 5 % (% en poids).

14. Milieu photochromique selon la revendication 13, dans lequel ledit chromophore actif est réparti de manière sensiblement homogène au sein dudit milieu photochromique.

15. Milieu photochromique selon la revendication 13 ou 14, dans lequel ledit chromophore actif est réparti de manière sensiblement non-homogène au sein de ladite substance support.

16. Milieu photochromique selon la revendication 13 ou 14, dans lequel ledit chromophore actif est réparti en portions individualisées de forte concentration, séparées par des portions dont la concentration en ledit chromophore actif est nulle ou insignifiante.

17. Milieu photochromique selon la revendication 16, dans lequel ledit chromophore actif est réparti en amas espacés, formés chacun par lesdites portions individualisées de forte concentration.

18. Milieu photochromique selon l'une quelconque des revendications 13 à 17, dans lequel ledit chromophore actif est lié chimiquement au polymère.

19. Milieu photochromique selon la revendication 18, qui est un copolymère comprenant un monomère chromophore actif polymérisable copolymérisé avec un monomère.

20. Milieu photochromique selon la revendication 18, qui est un polymère comprenant un monomère chromophore actif polymérisable.

21. Milieu photochromique pour le stockage de données, comprenant une matrice polymère selon l'une quelconque des revendications 1 à 7.

22. Système de stockage de données optiques tridimensionnel, comprenant un milieu photochromique selon l'une quelconque des revendications 8 à 21.

23. Procédé de facilitation de l'inscription de données dans un système de stockage de données optiques tridimensionnel et de diminution de la perte de données au cours du stockage ou de la lecture ultérieure des données, comprenant l'utilisation d'un milieu photochromique selon la revendication 21.
